# EUROPEAN PATENT APPLICATION

(11) **EP 4 718 376 A1**
(43) Date of publication of application: **01.04.2026**
(21) Application number: 24810670.0
(22) Date of filing: 11.03.2024
(51) Int. Cl.: G06T 7/00, G06T 7/70, G08B 21/02, G08B 25/00, G08B 25/04, H04N 7/18

(54) **FALL DETECTION SYSTEM, FALL DETECTION METHOD, AND FALL DETECTION PROGRAM**

(30) Priority: 23.05.2023 JP 2023084390
(71) Applicant: Konica Minolta, Inc., Tokyo 100-7015 (JP)
(72) Inventor: YOSHIZAWA, Masanori, Tokyo 100-7015 (JP)
(74) Representative: MFG Patentanwälte Meyer-Wildhagen Meggle-Freund Gerhard PartG mbB
(86) International application number: PCT/JP2024/009274
(87) International publication number: WO 2024/241662

(57) **Abstract**

Provided are a fall detection system, a fall detection method, and a fall detection program that can maintain accuracy in detection of fall or tumble of a monitoring target person, regardless of a place where monitoring is performed and a size of a region to be monitored. The fall detection system includes a video acquirer 310, a skeleton detector 320, a skeleton angle calculator 330, a primary fall determiner 350, and a secondary fall determiner 360. The video acquirer 310 acquires a video obtained by capturing an image of an object. The skeleton detector 320 detects skeleton information of the object shown in the video. The skeleton angle calculator 330 calculates an angle formed by a skeleton of the object with respect to a reference direction, based on the skeleton information of the object. The primary fall determiner 350 determines that the object is in a "temporary" fall state when the angle is within a range defined in advance for each skeleton. The secondary fall determiner 360 determines that the object is in a fall state when the determination that the object is in the "temporary" fall state continues for a predetermined first period of time.

## Description

### Technical Field

The present invention relates to a fall detection system, a fall detection method, and a fall detection program.

### Background Art

In recent years, monitoring cameras have been installed in various places for the purpose of ensuring the safety of passersby who pass along streets and users who use facilities. For example, in a nursing or care facility such as a hospital or a welfare facility for elders who need care, a nursing target person who is an inpatient or a care target person who is a resident may fall when he or she is walking in the nursing or care facility. A technique has been developed for estimating a posture of a nursing or care target person, based on a video of the nursing or care target person, in order to make it possible for a nurse, a caregiver, or the like to rush to the nursing or care target person immediately if the nursing or care target person falls (for example, Patent Literatures 1 and 2 listed below).

### Citations List

### Patent Literature

Patent Literature 1: JP 2020-034960 A
Patent Literature 2: JP 2020-052867 A

### Summary of Invention

### Technical Problem

According to the technique of Patent Literature 1 described above, the posture (standing posture, lying posture, or sitting posture) of a monitoring target person is estimated by maximum likelihood estimation, and whether the monitoring target person falls or tumbles is determined based on the estimated posture. According to the technique of Patent Literature 2 described above, the posture of a watching target person is estimated by using a database for inference generated by machine-learning with a correspondence relation between a change pattern of an angle of each joint of the watching target person and each posture. However, according to the techniques of Patent Literatures 1 and 2, when monitoring is performed at a place other than a site where learning has been performed, accuracy in estimation of a posture is significantly reduced, and accuracy in detection of fall or tumble of a monitoring target person is also reduced. Therefore, the techniques of Patent Literatures 1 and 2 have a problem of generalization.

With regard to safety monitoring in a manufacturing factory or the like, a region to be monitored is larger than that in a nursing or care facility or the like, and the amount of video data required for learning may be enormous. As a result, it is necessary to perform inefficient tuning for each site where monitoring is performed. Therefore, the techniques of Patent Literatures 1 and 2 have a problem that they are not practical.

The present invention has been devised in order to solve the problems described above. That is, a main objective of the present invention is to provide a fall detection system, a fall detection method, and a fall detection program that can maintain accuracy in detection of fall or tumble of a monitoring target person, regardless of a place where monitoring is performed and a size of a region to be monitored.

### Solution to Problem

The objective of the present invention is achieved by the following means.
(1) A fall detection system including: a video acquirer that acquires a video obtained by capturing an image of an object; a skeleton detector that detects skeleton information of the object shown in the video; a skeleton angle calculator that calculates an angle formed by a skeleton of the object with respect to a reference direction, based on the skeleton information of the object; a primary fall determiner that determines that the object is in a "temporary" fall state when the angle is within a range defined in advance for each skeleton; and a secondary fall determiner that determines that the object is in a fall state when the determination that the object is in the "temporary" fall state continues for a predetermined first period of time.
(2) The fall detection system according to (1), wherein the angle is an angle formed by at least one of a skeleton of a body part of the object and a skeleton of a leg part of the object with respect to the reference direction.
(3) The fall detection system according to (1) or (2), further including a tracking processor that holds same ID information for a same human over time, based on the skeleton information of the object.
(4) The fall detection system according to (1) or (2), wherein the skeleton detector estimates a certainty factor as to the skeleton information of the object, and the skeleton angle calculator calculates the angle when the skeleton information has the certainty factor more than or equal to a predetermined value.
(5) The fall detection system according to (1) or (2), wherein the primary fall determiner or the secondary fall determiner determines that the object is not in the fall state when the angle satisfies a preset condition although the angle is within the range defined in advance for each skeleton.
(6) The fall detection system according to (1) or (2), wherein, with regard to the object determined to be in the fall state, when the primary fall determiner subsequently determines that the object is not in the "temporary" fall state for a predetermined second period of time, the secondary fall determiner cancels the determination that the object is in the fall state.
(7) The fall detection system according to (1) or (2), further including: an imaging apparatus that captures an image of the object and outputs a video including a plurality of the images; a display device that displays the video shot by the imaging apparatus; and a display controller that controls the display device, wherein the skeleton detector calculates the skeleton information of the object by analyzing an image acquired by the video acquirer, and the display controller causes the display device to display the skeleton information of the object together with the video.
(8) The fall detection system according to (7), wherein the display controller makes a color of the object determined to be in the fall state by the secondary fall determiner or a color of the skeleton of the object different from a color of the object not determined to be in the fall state in the video to allow a user to visually distinguish between the object determined to be in the fall state and the object not determined to be in the fall state with ease.
(9) The fall detection system according to (7), wherein the display controller adds a mark to the object determined to be in the fall state by the secondary fall determiner in the video to allow a user to visually distinguish between the object determined to be in the fall state and the object not determined to be in the fall state with ease.
(10) The fall detection system according to (9), wherein the display controller keeps the mark added to the object determined to be in the fall state by the secondary fall determiner even when the determination that the object is in the fall state is canceled subsequently.
(11) The fall detection system according to (7), wherein the object is a human, and the display controller subjects the video to obscuring processing or blurring processing at a position of the object so as to prevent the object from being identified, based on position information included in the skeleton information of the object.
(12) The fall detection system according to (11), wherein the display controller displays the video of the object subjected to the obscuring processing or the blurring processing, with a graphic indicative of the skeleton information of the object superimposed on the video.
(13) The fall detection system according to (1) or (2), further including a fall notifier that provides a notification of fall of the object when the secondary fall determiner determines that the object is in the fall state.
(14) The fall detection system according to (13), further including a warning device that outputs an alarm by at least one of sound and light in accordance with the notification from the fall notifier.
(15) The fall detection system according to (1) or (2), further including a valid area inputter that receives input of a valid area where a determination result of the fall state of the object is valid, wherein the primary fall determiner determines the fall state of the object when position coordinates of an articulation point on at least one of a leg part, a head part, and a body part of the object are within the valid area.
(16) The fall detection system according to (15), further including: an imaging apparatus that captures an image of the object and outputs a video including a plurality of the images; a display device that displays the video shot by the imaging apparatus; and a display controller that causes the display device to display a graphic indicative of the valid area together with the video shot by the imaging apparatus.
(17) The fall detection system according to (1) or (2), further including an invalid area inputter that receives input of an invalid area where a determination result of the fall state of the object is invalid, wherein the primary fall determiner does not determine the fall state of the object when position coordinates of an articulation point on at least one of a leg part, a head part, and a body part of the object are within the invalid area.
(18) The fall detection system according to (17), further including: an imaging apparatus that captures an image of the object and outputs a video including a plurality of the images; a display device that displays the video shot by the imaging apparatus; and a display controller that causes the display device to display a graphic indicative of the invalid area together with the video shot by the imaging apparatus.
(19) A fall detection method including: a step (a) of acquiring a video obtained by capturing an image of an object; a step (b) of detecting skeleton information of the object shown in the video; a step (c) of calculating an angle formed by a skeleton of the object with respect to a reference direction, based on the skeleton information of the object; a step (d) of determining that the object is in a "temporary" fall state when the angle is within a range defined in advance for each skeleton; and a step (e) of determining that the object is in a fall state when the determination that the object is in the "temporary" fall state continues for a predetermined first period of time.
(20) A fall detection program for causing a computer to execute processing including: a step (a) of acquiring a video obtained by capturing an image of an object; a step (b) of detecting skeleton information of the object shown in the video; a step (c) of calculating an angle formed by a skeleton of the object with respect to a reference direction, based on the skeleton information of the object; a step (d) of determining that the object is in a "temporary" fall state when the angle is within a range defined in advance for each skeleton; and a step (e) of determining that the object is in a fall state when the determination that the object is in the "temporary" fall state continues for a predetermined first period of time.

### Advantageous Effects of Invention

According to the present invention, it is possible to maintain accuracy in detection of fall or tumble of an object, regardless of a place where monitoring of the object is performed and a size of a region to be monitored. That is, the robustness of the accuracy in detection of fall or tumble is improved.

### Brief Description of Drawings

Fig. 1 is a diagram exemplifying a schematic configuration of a fall detection system according to an embodiment of the present invention.
Fig. 2 is a block diagram exemplifying a schematic configuration of an imaging apparatus illustrated in Fig. 1.
Fig. 3 is a schematic diagram exemplifying a method of acquiring a video of a human.
Fig. 4 is a schematic block diagram exemplifying a hardware configuration of a fall detection apparatus illustrated in Fig. 1.
Fig. 5 is a flowchart exemplifying a processing procedure of a fall detection method by the fall detection system according to the embodiment.
Fig. 6 is a schematic diagram exemplifying detection of skeleton information.
Fig. 7 is a schematic diagram exemplifying calculation of a skeleton angle.
Fig. 8 is a schematic diagram exemplifying differentiation between a human in a fall state and a human in a non-fall state in a video.
Fig. 9 is a schematic diagram exemplifying privacy protection for a human.
Fig. 10 is a schematic diagram exemplifying a method of setting a valid area.
Fig. 11 is a schematic diagram explaining fall detection in the valid area.
Fig. 12 is a schematic diagram exemplifying a method of setting a valid area.
Fig. 13 is a schematic diagram explaining fall detection in an invalid area.

### Description of Embodiments

A fall detection system, a fall detection method, and a fall detection program according to an embodiment of the present invention will be described below with reference to the drawings. Note that in the drawings, the same components are denoted by the same reference signs, and the redundant description is omitted. In addition, dimensional ratios in the drawings are exaggerated for convenience of the description and may be different from actual ratios.

### (Embodiment)

A fall detection system according to the present embodiment detects fall or tumble of an object, based on skeleton information detected from a video obtained by capturing an image of the object. The skeleton information may include information about a plurality of articulation points on the object and information about connection between the articulation points. The information about each articulation point may be two-dimensional or three-dimensional position coordinates of the articulation point. Further, the information about the connection between the articulation points may be represented by, for example, a line segment connecting the articulation points. The object may be, for example, a skeleton detectable object such as a human or an animal. Hereinafter, a case where the object is a human will be mainly described, but the present invention can be similarly applied to a case where the object is an animal.

Further, in the present specification, the term "fall" means that a human falls on a place where he or she is present, on a floor surface or the like. The term "tumble" means, for example, that a human tumbles off a place such as a bed toward a lower place. Hereinafter, in the present specification, a case of detecting fall of a human will be mainly described, but the present invention can be similarly applied to a case of detecting tumble of a human.

### <Overall Configuration of Fall Detection System 100>

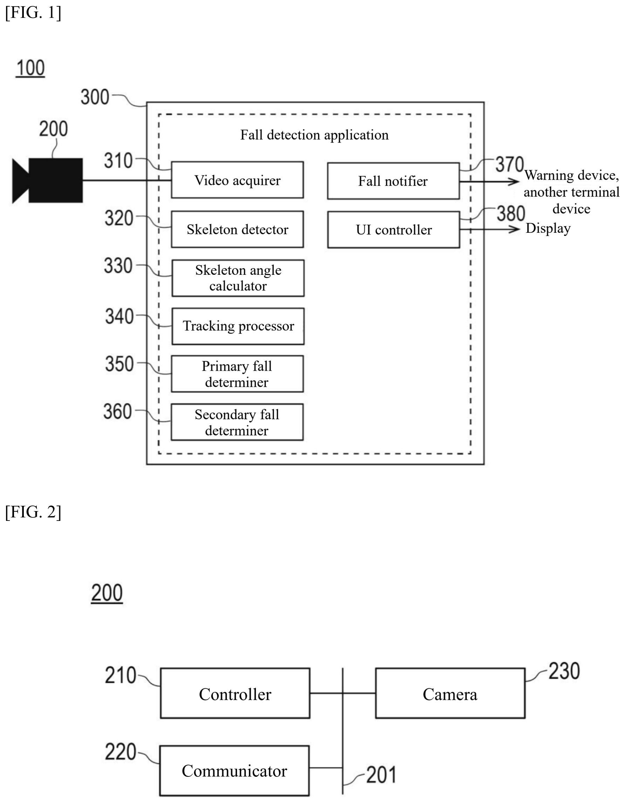

Fig. 1 is a block diagram exemplifying a schematic configuration of a fall detection system 100 according to an embodiment. The fall detection system 100 includes, for example, an imaging apparatus 200 and a fall detection apparatus 300. The imaging apparatus 200 is connected to the fall detection apparatus 300 via a communication network in a mutually communicatable manner.

The fall detection apparatus 300 may be, for example, a personal computer, a server, a smartphone, a tablet terminal, or the like.

### <Imaging Apparatus 200>

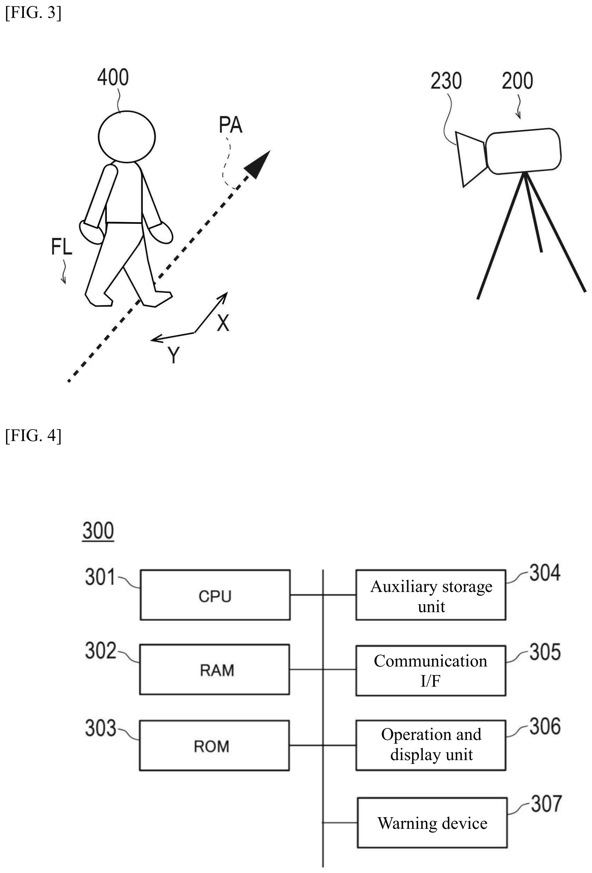

Fig. 2 is a block diagram exemplifying a schematic configuration of the imaging apparatus 200 illustrated in Fig. 1. The imaging apparatus 200 includes a controller 210, a communicator 220, and a camera 230, which are connected to one another by a bus 201. Fig. 3 is a schematic diagram exemplifying a method of acquiring a video of a human.

As illustrated in Fig. 3, the imaging apparatus 200 is installed at a place where at least a part of a movement path PA of a human 400 on a floor surface FL falls within a field of view of the camera 230. In Fig. 3, a depth direction of a video shot by the camera 230 of the imaging apparatus 200 (an optical axis direction of a lens of the camera 230) is defined as a Y direction, and a horizontal direction orthogonal to the depth direction is defined as an X direction.

For example, in a case where the imaging apparatus 200 detects fall of a worker engaged in work in a factory of an enterprise, the imaging apparatus 200 is installed at a place where at least a part of a movement path PA of the worker falls within the field of view of the camera 230. Furthermore, in a case where the imaging apparatus 200 detects fall of a nursing or care target person in a nursing or care facility, the imaging apparatus 200 is installed at a place where a room of the nursing or care target person, a corridor in the facility, or the like falls within the field of view of the camera 230.

The controller 210 includes, for example, a central processing unit (CPU) and a memory. Examples of the memory may include a random access memory (RAM) and a read-only memory (ROM). The CPU controls each component of the imaging apparatus 200 and performs arithmetic processing, in accordance with a fall detection program stored in the memory.

The controller 210 transmits a plurality of captured images (frames) obtained in such a manner that the camera 230 captures an image of a predetermined region, to the fall detection apparatus 300 via the communicator 220. The predetermined region is a three-dimensional region including the movement path of the object.

The communicator 220 includes, for example, an interface circuit for communicating with the fall detection apparatus 300 or the like via a communication network.

The camera 230 may be, for example, a standard camera (bullet camera) used as a security camera or a monitoring camera. The camera 230 may be a wide-angle camera (e.g., a ceiling-mounted 360-degree camera) having a wider angle of view than that of a standard camera. In the following description, in order to simplify the description, it is assumed that the camera 230 is a standard camera.

It is desirable that the imaging apparatus 200 previously adjusts the position, the lens distortion, and the like of the camera 230 so as to capture an image of a human in a substantially vertical direction when the human is in a standing position. Specifically, in a case where the camera 230 is a bullet camera, the camera 230 may be arranged so as to capture an image of the human from the side or obliquely from above. Furthermore, in a case where a ceiling-mounted 360-degree camera is used as the camera 230, the lens distortion and the viewpoint may be adjusted so that the angle of view is the same as that in a case where the above-described bullet camera captures an image.

The camera 230 may be, for example, a visible light camera. The visible light camera may capture an image of a predetermined region by receiving, with a complememtary metal oxide semiconductor (CMOS) sensor, reflected light of a visible ray reflected off an object in the predetermined region.

Alternatively, the camera 230 may be configured using a near-infrared camera instead of the visible light camera. The infrared camera may capture an image of a predetermined region by irradiating, by a light emitting device (LED), the predetermined region with a near-infrared ray and receiving, by the CMOS sensor, reflected light of the near-infrared ray reflected off an object in the predetermined region. In this case, the image captured by the camera 230 may be a monochrome image in which each pixel represents the reflectance of a near-infrared ray.

The imaging apparatus 200 may capture an image of a predetermined region as a moving image including a plurality of temporally consecutive captured images (frames) at a frame rate between 15 fps and 30 fps, for example. Furthermore, the camera 230 may be configured using a near-infrared camera and a visible light camera in combination.

Note that some or all of the functions of the imaging apparatus 200 may be executed by the fall detection apparatus 300 or another information processing apparatus (not illustrated).

### <Fall Detection Apparatus 300>

Fig. 4 is a schematic block diagram exemplifying a hardware configuration of the fall detection apparatus 300 illustrated in Fig. 1. The fall detection apparatus 300 includes, for example, a CPU 301, a RAM 302, a ROM 303, an auxiliary storage unit 304, a communication I/F 305, and an operation and display unit 306. The constituent elements of the fall detection apparatus 300 are mutually connected by an internal bus.

The auxiliary storage unit 304 may be configured using, for example, a solid state drive (SSD), a hard disk drive (HDD), or the like. The auxiliary storage unit 304 stores, for example, a program such as a fall detection program, a video obtained by capturing a predetermined region including a human, a date and time at which the video is shot, conditions concerning fall, and the like. The conditions concerning fall include a fall condition under which a primary fall determiner 350 described later determines that a human has fallen, and an exceptive condition under which the primary fall determiner 350 does not determine exceptionally that the human has fallen even when the fall condition is satisfied. Details of the conditions concerning fall will be described later.

The communication I/F 305 is an interface circuit for communicating with, for example, another apparatus such as the imaging apparatus 200 via a network. The interface circuit complies with standards of a local area network (LAN), a universal serial bus (USB), a mobile industry processor interface (MIPI), and the like.

The operation and display unit 306 includes an input unit and an output unit. The input unit includes, for example, a touch panel, a keyboard, a mouse, and the like. The input unit is used by a user to perform touch operation with the touch panel, character input with the keyboard, the mouse, and the like, various settings, and the like. The output unit includes a display (display device) and displays, for example, a video showing an object and shot by the imaging apparatus 200.

The warning device 307 outputs an alarm in accordance with a human fall notification from a fall notifier 370 (see Fig. 1). The warning device 307 includes, for example, a speaker, and can output a warning sound or voice as an alarm to the speaker. The warning device 307 also includes a lamp or a light emitting diode (LED), and can turn on or blink the lamp or the LED as an alarm.

### <Function of Fall Detection Apparatus 300>

Next, with reference to Fig. 1 again, the function (fall detection application) of the fall detection apparatus 300 will be described. The fall detection apparatus 300 functions as, for example, a video acquirer 310, a skeleton detector 320, a skeleton angle calculator 330, a tracking processor 340, the primary fall determiner 350, a secondary fall determiner 360, the fall notifier 370, and a UI controller 380. These functions are implemented in such a manner that the CPU 301 executes the fall detection program.

### [Video Acquisition Processing]

The video acquirer 310 acquires a video obtained by capturing an image of a predetermined region including a human. The video may be, for example, a plurality of the captured images corresponding to the number of frames showing the predetermined region including the human and obtained by the imaging apparatus 200. The video acquirer 310 receives, for example, a video from the imaging apparatus 200 via the communication I/F 305 and acquires an image. The acquired image is decoded, and a two-dimensional image (snapshot) that is a still image is obtained.

Furthermore, the video acquirer 310 performs predetermined preprocessing on the acquired two-dimensional image and inputs the preprocessed image data to the skeleton detector 320. The preprocessing may be, for example, convolution integration (smoothing, sharpening), tone correction using a one-dimensional lookup table, or the like. By performing the preprocessing on the two-dimensional image, even if there is a portion in the video in which the human is difficult to visually recognize, the human is highlighted, so that the accuracy in detection of the skeleton information by the skeleton detector 320 is improved.

In a case where the video shot by the imaging apparatus 200 is previously stored in the auxiliary storage unit 304 or the like, the video acquirer 310 can also acquire the video by reading the video from the auxiliary storage unit 304 or the like. The video shot by the imaging apparatus 200 may be stored in an external storage device or the like, and the video acquirer 310 may be configured to read the video from the external storage device. Alternatively, the video shot by the imaging apparatus 200 may be recorded in a recording medium such as a USB memory, and the video acquirer 310 may acquire the video offline by reading the video from the recording medium.

### [Human Skeleton Detection Processing]

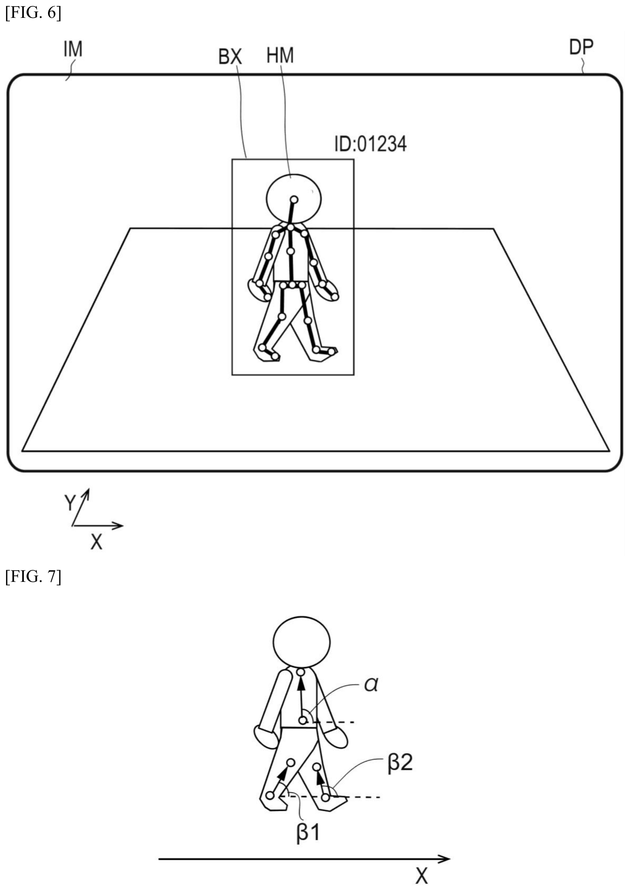

The skeleton detector 320 analyzes the image acquired by the video acquirer 310 and detects (estimates) skeleton information of the human shown in the video. The skeleton information may include position information on a plurality of articulation points on an eye, a nose, a neck, a shoulder, an elbow, a wrist, a waist, a knee, an ankle, and the like which are skeleton feature points (key articulation points) on the human, and line segments each connecting two of the articulation points (see, for example, Fig. 6). The position information of each articulation point may be coordinates (an X coordinate and a Y coordinate) of the articulation point on the captured image. Note that in the present specification, descriptions of some articulation points may be omitted for convenience of illustration in the drawings. The number of humans may be one or more.

The skeleton detector 320 estimates the skeleton information using, for example, a trained model for detecting the articulation points on the human from a rectangle including the human (hereinafter, also referred to as a "human rectangle"). The skeleton detector 320 estimates the articulation points and the certainty factors (scores of the certainty of estimation) of the respective articulation points. For each articulation point, it is desirable to use articulation points more than or equal to a predetermined threshold value. This is because in a case where a certainty factor is low due to the nature of machine learning, there is a high possibility that a correct detection result is not obtained. The skeleton detector 320 outputs the skeleton information and the certainty factors to the skeleton angle calculator 330 and the tracking processor 340.

The human rectangle is a region including articulation points and nodes on a human in an image. As such a trained model, for example, models such as OpenPose (https://arxiv.org/abs/1812.08008), Deep Pose (https://arxiv.org/abs/1312.4659), and ResNet (https://arxiv.org/abs/1512.03385) are known.

### [Skeleton Angle Calculation Processing]

The skeleton angle calculator 330 calculates an angle formed by a skeleton of the human with respect to a reference direction (hereinafter, also referred to as a "skeleton angle") based on the skeleton information with regard to each human. In the present specification, the skeleton corresponds to, for example, a line connecting a predetermined articulation point and an articulation point or a line connecting an articulation point and a central point between two articulation points. The predetermined articulation point includes, for example, at least one of a body part and a leg part of the human. The reference direction may be set to, for example, the horizontal direction (the X direction in Fig. 3). Details of the calculation of the skeleton angle by the skeleton angle calculator 330 will be described later.

In the present embodiment, in order to ensure the reliability of the calculated skeleton angle, the skeleton angle calculator 330 calculates the skeleton angle in a case where the articulation points each have a certainty factor more than or equal to a predetermined value.

### [Human Tracking Processing]

The tracking processor 340 chronologically identifies a human by holding the same identification (ID) information about the same human over time, based on the skeleton information of the human. The tracking processor 340 assigns unique ID information to each of a plurality of humans shown in a video, and holds the same ID information for the same human. More specifically, the tracking processor 340 obtains a degree of proximity of the position information of the articulation points from the skeleton information of the human for the latest captured image and the previous captured image, and associates the degree of proximity with the ID information of the human to be tracked.

Furthermore, although the case where the coordinate information detected by the skeleton detection is used has been exemplified, the position information of the human rectangle may be used. In a case where means for detecting a human by object detection or the like can be used separately, the latest captured image and the previous captured image may be associated with the ID information of the human to be tracked, by using the degree of approximation between image data items of region portions in which the human has been detected. Since these tracking processing methods are known technologies, the detailed description thereof is omitted.

### [Primary Fall Determination Processing]

When the calculated skeleton angle of each human is within a predetermined range defined in advance for each skeleton, the primary fall determiner 350 determines that the human is in a fall state. In the present specification, the fall state of the human determined by the primary fall determiner 350 is referred to as a "temporary" fall state.

For example, as fall conditions, in a case where the skeleton angle is within a predetermined range for each skeleton as shown in Table 1 below, the primary fall determiner 350 determines that the human is in the fall state. Note that the fall conditions listed in the table are merely examples, and the fall conditions are not limited to these angle ranges. It is desirable that the fall conditions are appropriately changed (adjusted) in accordance with, for example, installation conditions of the imaging apparatus 200. Hereinafter, the determination result by the primary fall determiner 350 is also referred to as a "primary fall determination result".

**[Table 1]**

| No. | Fall Conditions | |
|---|---|---|
| 1 | Skeleton angle (α) of body part | 0 to 30 degrees, 150 to 360 degrees |
| 2 | Skeleton angle (β) of leg part | 0 to 5 degrees, 175 to 360 degrees |

More specifically, the primary fall determiner 350 may determine that the human is in the fall state when at least one of No. 1 and No. 2 in Table 1 is satisfied. For example, the skeleton of the body part is a line connecting the articulation point on the waist part and the articulation point on the neck part, and the skeleton angle of the body part is an angle α formed by the skeleton of the body part with respect to the reference direction (see Fig. 7). The present inventor has experimentally confirmed that, when a human has fallen, the skeleton angle (α) of the body part is generally within a range of 0 to 30 degrees or a range of 150 to 360 degrees with respect to the horizontal direction.

The skeleton of the leg part is a line connecting the articulation point on the foot (or ankle) and the articulation point on the knee, and the skeleton angle of the leg part is an angle (β) formed by the skeleton of the leg part with respect to the reference direction. The skeleton angle β of the leg part includes a skeleton angle β1 of the right leg and a skeleton angle β2 of the left leg (see Fig. 7). The present inventor has experimentally confirmed that, when a human has fallen, the skeleton angle (β) of the leg part is generally within a range of 0 to 5 degrees or a range of 175 to 360 degrees with respect to the horizontal direction.

That is, from the detected skeleton information, when the skeleton angle (α) of the body part is in the range of 0 to 30 degrees or in the range of 150 to 360 degrees and the skeleton angle (β) of the leg part is in the range of 0 to 5 degrees or in the range of 175 to 360 degrees, it can be said that the possibility that the human has fallen is high. When one of the skeleton angle (α) of the body part and the skeleton angle (β) of the leg part satisfies the corresponding fall condition, the human may have fallen.

As described above, the fall determination by the primary fall determiner 350 is determined only by whether the skeleton angle of the human is within the predetermined range defined in advance for each skeleton. Therefore, in addition to the fall, a case where the human temporarily takes a posture similar to fall may also be determined as the "temporary" fall state. Therefore, the "temporary" fall state is not a formal fall state of the human, but indicates that the human may have fallen.

### [Secondary Fall Determination Processing]

The secondary fall determiner 360 stores the primary fall determination result of the "temporary" fall state in the RAM 302 in association with the ID information and the number of the corresponding captured image. Through the processing of associating the primary fall determination result with the ID information and the number of the captured image, the primary fall determination result of the human is accumulated as a history in the RAM 302. Note that this processing may be configured to be performed by the primary fall determiner 350.

The secondary fall determiner 360 determines that the human is in the fall state when the determination that the human is in the "temporary" fall state continues for a predetermined first period of time. Specifically, the secondary fall determiner 360 determines that the human is in the fall state when the primary fall determination result for the human having the same ID information is determined as the "temporary" fall state even after the first period of time elapses. The first period of time may be set to a period of time sufficiently longer than a period of time of an action that a human temporarily performs in a daily life, for example, an action such as temporarily "squatting" to pick up something dropped on the floor. That is, when the "temporary" fall state of the human continues for a period of time longer than an action similar to fall that the human temporarily performs in daily life, the secondary fall determiner 360 determines that the human is in the "formal" fall state.

The duration of the determination that the human is in the "temporary" fall state can be calculated by, for example, dividing, by a frame rate, the number of consecutive frames in a period in which the determination that the human is in the "temporary" fall state continues. The value of the first period of time may be input by the user through the operation and display unit 306, for example. Alternatively, the value of the first period of time can be previously stored in the auxiliary storage unit 304.

Note that instead of calculating the duration using the number of consecutive frames, the secondary fall determiner 360 may be configured to include a timer that measures the duration of the determination that the human is in the "temporary" fall state.

Furthermore, instead of calculating the duration, the secondary fall determiner 360 may be configured to determine that the human is in the fall state when the number of frames in which the determination that the human is in the "temporary" fall state continues is a predetermined number. The predetermined number may be input by the user through the operation and display unit 306, for example. Alternatively, the predetermined number can be stored in the auxiliary storage unit 304.

### [Fall Notification Processing]

When the secondary fall determiner 360 determines that the human is in the fall state, the fall notifier 370 notifies the warning device 307 of the fall of the human. The fall notifier 370 can also notify another terminal device (not illustrated) of the fall of the human by communication via a network. For example, the fall notifier 370 can transmit an e-mail with a video (image) of the human who has fallen to another terminal device. The image to be transmitted may be either a captured image or an image obtained by processing an original captured image, such as an image obtained by superimposing skeleton information on a captured image. Adding an image to the notification allows a person who has received the notification to know the situation of the site where the human 400 is present in more detail, which is more effective in supporting the human 400.

In addition, the fall notifier 370 can transmit a video/message to another terminal device via a social networking service (SNS) or by using a push notification. By asking a supportable member for support using an e-mail, the warning device 307, or the like, appropriate support corresponding to the situation can be immediately provided to the fallen human. Further, if the supportable member has a terminal capable of receiving the notification from the fall notifier 370, the supportable member can know a necessity of a countermeasure even from a remote place.

Furthermore, the fall notifier 370 may be configured to notify a system different from the fall detection system 100, such as a core system, of the fall of the human 400 and record the fall. Thus, for example, it is possible to save a record of the fall of the human 400 for the purpose of personal use for the human 400. Alternatively, the fall notifier 370 can transmit the fall of the human 400 to a database system intended for commercial, medical, academic, or other use, and accumulate, as data, the skeleton information of the human whose fall has been detected.

### [Display Processing]

The UI controller 380 functions as a display controller, and displays the video acquired by the video acquirer 310 on the display. The UI controller 380 can also display the articulation points on the human and the line segments each connecting two of the articulation points with the articulation points and the line segments superimposed on the human in the video.

Note that the imaging apparatus 200 or another information processing apparatus (not illustrated) may be configured to perform some or all of the functions of the fall detection apparatus 300.

### <Processing Procedure of Travel Detection Method>

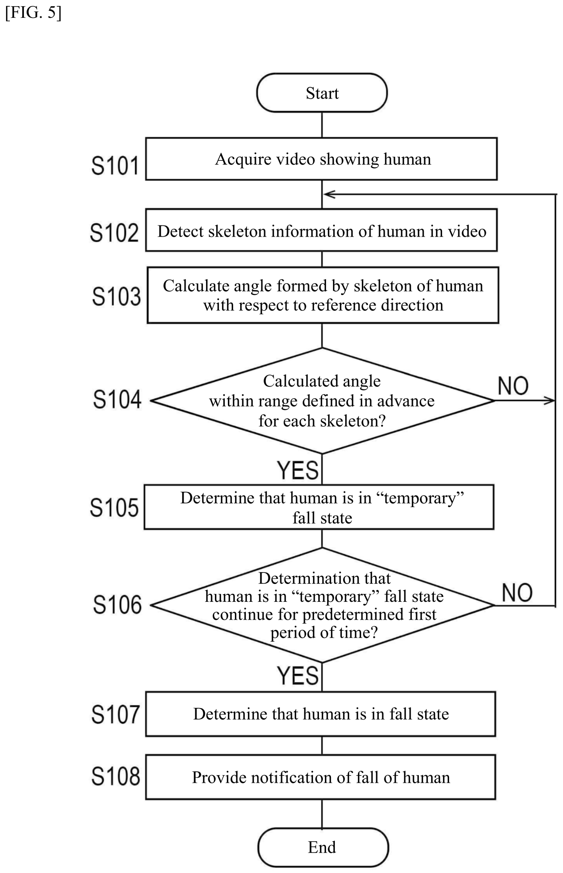

Fig. 5 is a flowchart exemplifying a processing procedure of a fall detection method by the fall detection system 100 according to the embodiment. Processing of the flowchart illustrated in Fig. 5 is implemented in such a manner that the CPU 301 executes the fall detection program. Fig. 6 is a schematic diagram exemplifying detection of the skeleton information, and Fig. 7 is a schematic diagram exemplifying calculation of the skeleton angle.

In the following description, it is assumed that an image of at least one human is captured by the camera 230. Note that for convenience of illustration, the drawings exemplify the case where an image of one human is captured, but an image of a plurality of humans may be captured.

As illustrated in Fig. 3, the imaging apparatus 200 is installed so as to be capable of capturing an image of the floor surface FL in the predetermined region and the human 400 on the floor surface FL. More specifically, the installation position of the imaging apparatus 200, as well as the orientation (the optical axis direction of the lens), the lens distortion, and the like of the camera 230 are adjusted such that the floor surface FL and the human 400 in the standing position fall within the field of view of the camera 230. For example, the imaging apparatus 200 is installed on the floor surface FL at a predetermined distance away from the position at which the human 400 is present. In addition, the imaging apparatus 200 is adjusted to direct the camera 230 slightly downward with respect to the horizontal direction such that the entire floor surface FL in the predetermined region falls within the field of view of the camera 230.

First, the video acquirer 310 acquires a video showing a human (step S101). For example, the imaging apparatus 200 captures an image of one human 400 moving in the predetermined region, and transmits a video obtained from the captured image of the human 400 to the video acquirer 310. The video acquirer 310 receives and acquires the video showing the human 400 from the imaging apparatus 200, and transmits the acquired video to the skeleton detector 320.

Next, the skeleton detector 320 detects skeleton information of the human shown in the video (step S102). The skeleton detector 320 sequentially extracts the captured images from the video acquired by the video acquirer 310 and detects the skeleton information of the human 400. When a plurality of humans are shown in the video, skeleton information is detected for each of the plurality of humans. The detected skeleton information is output to the skeleton angle calculator 330 and the tracking processor 340.

As illustrated in Fig. 6, the UI controller 380 causes the display DP to display the skeleton information of the human 400 together with the video IM acquired by the video acquirer 310. In Fig. 6, the human 400 in Fig. 3 is represented by the human HM in the video. Further, the UI controller 380 causes the display DP to display the human rectangle BX of the human HM whose skeleton information has been detected. The UI controller 380 causes the display DP to display the articulation points on the human HM and the line segments (skeleton) each connecting two of the articulation points from the skeleton information, with the articulation points and the line segments superimposed on the human HM shown in the video IM. In Fig. 6, hollow circles located at the eye, nose, neck, shoulder, elbow, wrist, waist, knee, ankle, and the like of the human HM correspond to the articulation points, and the black lines each connecting two of the hollow circles correspond to the line segments.

Furthermore, the tracking processor 340 holds the same ID information on the same human over time. For example, the tracking processor 340 assigns ID information (ID: 01234) to the human HM and holds the assigned ID information until the end of monitoring. Thus, even when a plurality of humans are shown in the video IM and the human HM stands up after falling down, the user can determine the fall of the human HM from the video. That is, it is possible to prevent or inhibit the human HM who has fallen from becoming indistinguishable from other humans who have not fallen since the human HM has stood up thereafter. As a result, the user can perform a necessary treatment such as verbal assistance or rescue on the human HM who fell.

Next, the skeleton angle calculator 330 calculates an angle formed by the skeleton of the human HM with respect to the reference direction, based on the skeleton information of the human HM (step S103). As illustrated in Fig. 7, the skeleton angle calculator 330 calculates the angle α formed by the line (skeleton) connecting the articulation point on the waist (the center of the waist part) and the articulation point on the neck (or the center of both shoulders) in the body part of the human HM, with respect to the reference direction. Further, the skeleton angle calculator 330 calculates each of the angles β1 and β2 formed by the lines (skeleton) connecting the articulation points on the feet (or ankles) and the articulation points on the knees in the right and left legs (leg part), with respect to the reference direction. When the certainty factor of the skeleton information is less than the threshold value, the skeleton angle calculator 330 skips the calculation of the skeleton angle for the human.

Next, the primary fall determiner 350 determines whether the calculated skeleton angle is within the range defined in advance for each skeleton (step S104). The primary fall determiner 350 determines, for example, whether the angle α is within the range of the angle of the body part in Table 1 and the angles β1 and β2 are within the range of the angle of the leg part in the same table.

When the calculated skeleton angle is not within the range defined in advance for each skeleton (step S104: NO), the primary fall determiner 350 proceeds to step S102, and the skeleton detector 320 detects skeleton information of the human for the next captured image. On the other hand, when the calculated skeleton angle is within the range defined in advance for each skeleton (step S104: YES), it is determined that the human HM is in the "temporary" fall state (step S105).

Next, the secondary fall determiner 360 determines whether the determination that the human HM is in the "temporary" fall state by the primary fall determiner 350 continues for the predetermined first period of time (step S106). When the duration of the determination that the human HM is in the "temporary" fall state does not reach the first period of time (step S106: NO), the processing proceeds to step S102, and the skeleton detector 320 detects skeleton information of the human for the next captured image.

On the other hand, when the duration of the determination that the human HM is in the "temporary" fall state reaches the first period of time (step S106: YES), the secondary fall determiner 360 determines that the human HM is in the fall state (step S107). Thus, even when the human HM temporarily becomes a nearly fallen posture, for example, the human HM squats to pick up something, it is possible to suppress an erroneous determination that the human HM is in the fall state.

Further, the secondary fall determiner 360 deletes, from the RAM 302, or invalidates the data of the determination result of the "temporary" fall state associated with the ID information of the human HM determined to be in the fall state. When the data of the determination result is deleted, a history of the determination result of the "temporary" fall state is not saved. On the other hand, when the data of the determination result of the "temporary" fall state is invalidated, the history of the determination result of the "temporary" fall state is saved, but is not used for the determination of the fall state by the secondary fall determiner 360. The secondary fall determiner 360 can also delete, from the RAM 302, or invalidate the data of the determination result of the "temporary" fall state associated with the ID information of the human whose "temporary" fall state is no longer continued.

Next, when the secondary fall determiner 360 determines that the human HM is in the fall state, the fall notifier 370 provides a notification of the fall of the human HM (step S108). The fall notifier 370 notifies the warning device 307 or another terminal device of the fall of the human HM through the network. For example, the warning device 307 outputs an alarm by audio output (sound) from the speaker and lighting (light) of the lamp, in accordance with the notification from the fall notifier 370.

Thus, it is possible to prompt a person around the human 400 who has fallen or a supportable member (such as a healthcare professional) to take an appropriate action for the human 400 at an early stage.

### [Differentiation between Human in Fall State and Human in Non-Fall State in Video]

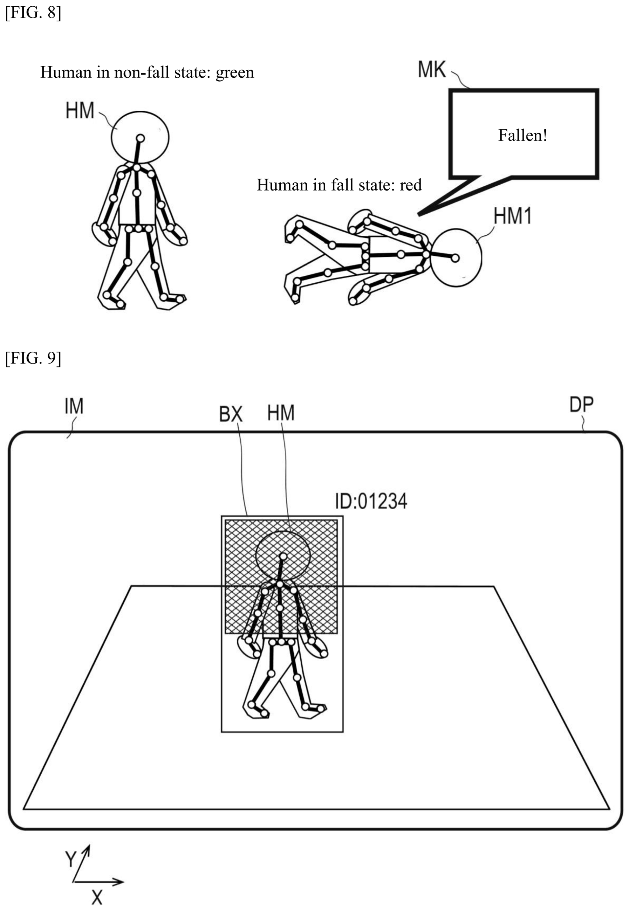

Fig. 8 is a schematic diagram exemplifying differentiation between a human HM1 in the fall state and a human HM in the non-fall state in a video. The UI controller 380 makes the color of the human HM1 determined to be in the fall state by the secondary fall determiner 360 different from the color of the human HM not determined to be in the fall state (the human in the non-fall state) in the video. The UI controller 380 changes the color of the human HM1 determined to be in the fall state to red and the color of the human HM not determined to be in the fall state to green, for example. Thus, the user can visually distinguish between the human HM1 determined to be in the fall state and the human HM not determined to be in the fall state with ease.

Alternatively, the UI controller 380 adds, in the video, a mark MK to the human HM1 determined to be in the fall state by the secondary fall determiner 360, but does not add the mark MK to the human HM not determined to be in the fall state. Thus, it is possible to differentiate between the fall state and the non-fall state of the human in the video. The mark MK may be a graphic and/or a character.

In addition, the UI controller 380 can also keep the color of the human HM1 different or keep the mark added to the human HM1 even when, with regard to the human HM1 determined to be in the fall state by the secondary fall determiner 360, the determination that the human HM1 is in the fall state is canceled subsequently.

In this way, by differentiating the human HM1 in the fall state from the human HM in the non-fall state in the video, the user can clearly grasp the human HM1 who has fallen (the human who needs treatment) from the video.

### [Privacy Protection for Human]

Fig. 9 is a schematic diagram exemplifying privacy protection for a human. For example, it is necessary to consider the privacy of a human in a case where a video is published or in a case where a human whose image is to be captured is reluctant to the act of capturing an image. As illustrated in Fig. 9, the UI controller 380 can perform predetermined processing on the video at the position of the human HM, based on the position information included in the skeleton information of the human HM so as to prevent the human shown in the video from being identified. The predetermined processing may be, for example, obscuring processing, blurring processing, or masking processing for a rectangular region covering the face, upper body, or entire body of the human HM. Thus, it is possible to monitor/watch over the human 400 while protecting the privacy of the human 400.

Furthermore, the UI controller 380 can also display the video showing the human HM and subjected to the predetermined processing, with a graphic indicative of the skeleton information of the human HM superimposed on the video. Thus, since the user can visually recognize the skeleton information of the human HM even when the predetermined processing is performed, the user can understand the posture of the human HM in the video with ease. As a result, the user can determine the posture of the human 400 who has fallen, with ease.

### [Exception to Primary Fall Determination Processing]

Even in a case where the skeleton angle is within the range defined in advance for each skeleton, when the skeleton angle satisfies a preset condition, the primary fall determiner 350 exceptionally determines that the human is not in the fall state. More specifically, for example, in a case where the skeleton angle (β) of the leg part satisfies the following condition in Table 2, the primary fall determiner 350 determines that the human HM is not in the fall state even when the skeleton angle (α) of the body part satisfies the fall condition. The skeleton angle (β) of the leg part in Table 2 corresponds to a posture of the human 400 who bends down in accordance with actions such as a bow, cleaning (with a mop or a vacuum cleaner), raising and lowering of a load, and a predetermined kind of work. These actions are normal actions that a human performs on a daily basis and are not an abnormal state such as fall; therefore, these actions are excluded from determination targets.

**[Table 2]**

| No. | Exceptive Condition | |
|---|---|---|
| 1 | Skeleton angle (β) of leg part | 75 to 105 degrees |

### [Exception to Secondary Fall Determination Processing]

Even in the case where the skeleton angle is within the range defined in advance for each skeleton, when the skeleton angle satisfies a preset condition, the secondary fall determiner 360 exceptionally determines that the human is not in the fall state. The preset condition is the same as that in the case of the primary fall determiner 350.

Providing the exceptions to the primary fall determination processing and secondary fall determination processing can suppress an erroneous determination even when, for example, a human bends down. In this way, in each of the primary fall determination processing and the secondary fall determination processing, it is possible to prevent or suppress false notification of fall even when a human takes a specific posture similar to fall.

### [Cancellation of Determination by Secondary Fall Determiner 360]

When the primary fall determiner 350 determines that the human determined to be in the fall state is not in the fall state for a predetermined second period of time, the secondary fall determiner 360 can cancel the determination that the human is in the fall state. As a result, the continuation of the fall state of the human can be accurately grasped. The value of the second period of time may be input by the user through the operation and display unit, for example. Alternatively, the value of the second period of time can be previously stored in the auxiliary storage unit 304.

In this way, the secondary fall determiner 360 makes a decision to cancel the determination of the fall state when the secondary fall determiner 360 detects that the user is not in the fall state consecutively for the predetermined second period of time. Thus, chattering in detection can be suppressed, and stable operation is enabled.

### [Detection in Valid Area]

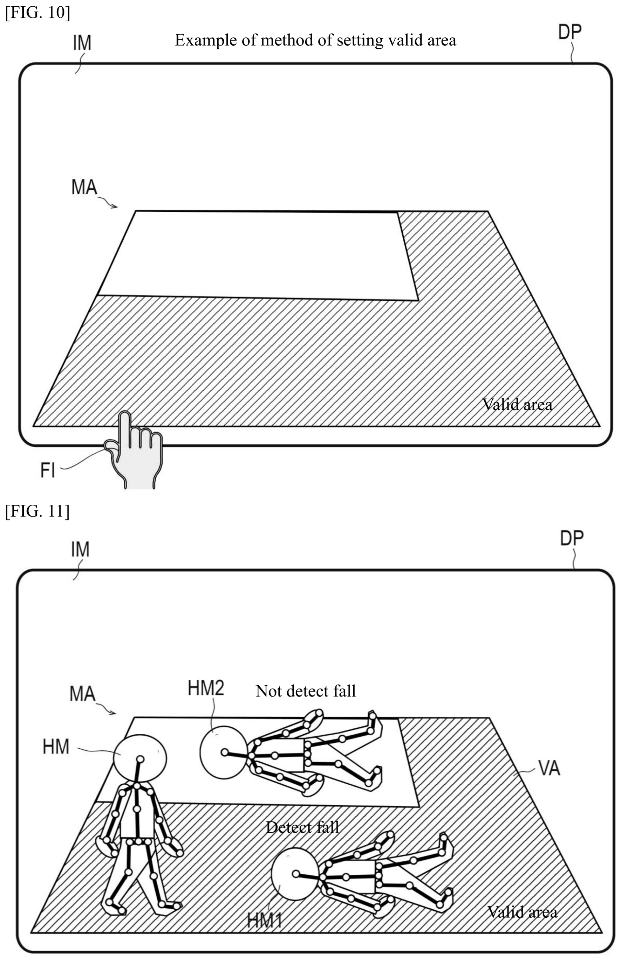

Fig. 10 is a schematic diagram exemplifying a method of setting a valid area. In Fig. 10, a shaded region corresponds to the valid area. Fig. 11 is a schematic diagram explaining fall detection in the valid area.

For example, the UI controller 380 allows the user to input the valid area (monitoring region) VA on the video through the operation and display unit 306. The operation and display unit 306 and the UI controller 380 function as a valid area inputter. For example, the input of the valid area VA may be performed to specify the range of the valid area VA by touching a touch panel with a finger FI or by using an input device such as a keyboard or a mouse. Alternatively, in a case where the imaging apparatus 200 is stationary, the value of the valid area VA may be previously stored in the auxiliary storage device.

The valid area VA is an area in which the determination result of the primary fall determiner 350 is valid when the position coordinates of the articulation point on at least one of the leg part, the head part, and the body part of the human HM are within the area. The valid area VA may have at least one or more polygonal shapes.

As illustrated in Fig. 11, with regard to the human HM1 who is in the valid area VA, a fall detection result by the primary fall determiner 350 is valid. On the other hand, with regard to the human HM2 who is not in the valid area VA (i.e., who is outside the valid area VA), a fall detection result by the primary fall determiner 350 is not valid. That is, the primary fall determiner 350 does not detect fall of the human HM2.

Thus, for example, only necessary fall detection can be performed by monitoring the valid area including an area of activity where the human may fall. Furthermore, in skeleton detection, it is possible to prevent or suppress erroneous detection, as a human, of equipment having a complicated structure with a portion structurally similar to a human (e.g., an industrial robot or a humanoid robot). As a result, it is possible to prevent or suppress false notification of detection of fall of a human.

In addition, the UI controller 380 displays a graphic indicative of the valid area VA on the display DP together with the video shot by the imaging apparatus 200. Thus, it becomes possible to intuitively understand which area is set as the valid area.

### [Detection in Invalid Area]

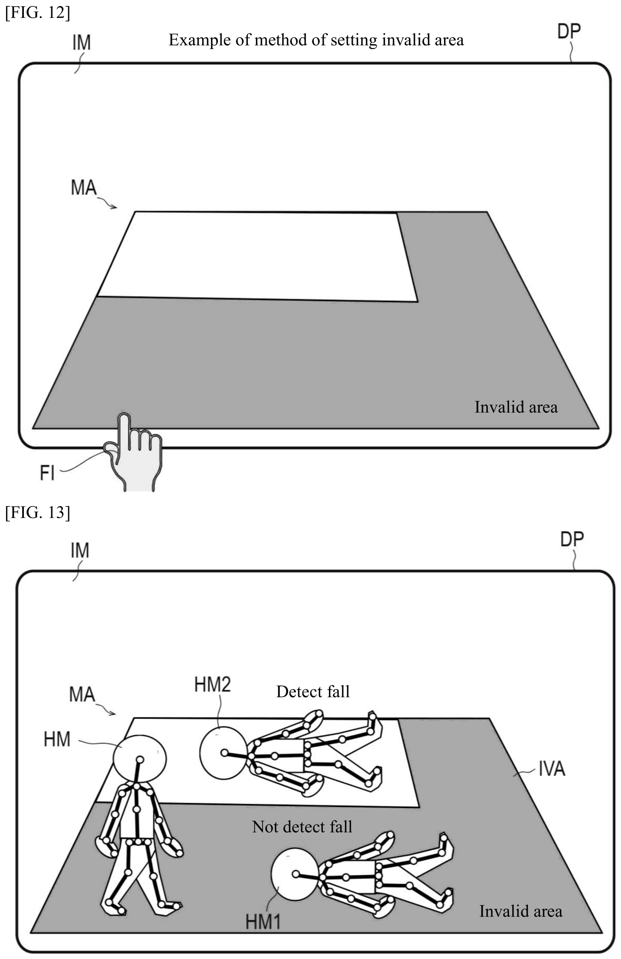

Fig. 12 is a schematic diagram exemplifying a method of setting an invalid area. In Fig. 12, a gray region corresponds to the invalid area. Fig. 13 is a schematic diagram explaining fall detection in the invalid area. For example, the UI controller 380 allows the user to input the invalid area (non-monitoring region) IVA on the video through the operation and display unit 306. The operation and display unit 306 and the UI controller 380 function as an invalid area inputter. Since the input of the invalid area IVA is the same as the input of the valid area VA, the detailed description thereof is omitted (see Fig. 10).

The invalid area IVA is an area in which the determination result of the primary fall determiner 350 is invalid when the position coordinates of the articulation point on at least one of the leg part, the head part, and the body part of the human HM are within the area. The invalid area IVA may have at least one or more polygonal shapes.

As illustrated in Fig. 13, with regard to the human HM1 who is in the invalid area IVA, a fall detection result by the primary fall determiner 350 is invalid. On the other hand, with regard to the human HM2 who is not in the invalid area IVA (i.e., who is outside the invalid area IVA), a fall detection result by the primary fall determiner 350 is not invalid. That is, the primary fall determiner 350 detects fall of the human HM2.

Thus, for example, only necessary fall detection can be performed by monitoring the invalid area including a portion outside an area of activity, where the human does not fall. Furthermore, in skeleton detection, it is possible to prevent or suppress erroneous detection, as a human, of equipment having a complicated structure with a portion structurally similar to a human (e.g., an industrial robot or a humanoid robot). As a result, it is possible to prevent or suppress false notification of detection of fall of a human.

In addition, the UI controller 380 displays a graphic indicative of the invalid area IVA on the display together with the video shot by the imaging apparatus 200. Thus, it becomes possible to intuitively understand which area is set as the invalid area (non-monitoring region).

According to the present embodiment described above, when the angle formed by the skeleton of the human 400 with respect to the reference direction is within the range defined in advance for each skeleton, it is determined that the human 400 is in the "temporary" fall state. Then, when the determination that the human 400 is in the "temporary" fall state continues for the predetermined first period of time, it is determined that the human is in the fall state. Therefore, it is possible to maintain accuracy in detection of fall or tumble of a human, regardless of a place where monitoring of the human is performed and a size of a region to be monitored. That is, the robustness of the accuracy in detection of fall or tumble is improved.

The configuration of the fall detection system 100 described above is the main configuration for describing the features of the above-described embodiment, and the present invention is not limited to the above-described configuration and can be variously modified within the scope of the claims. Further, the present invention does not exclude a configuration of a general fall detection system.

For example, although the case of detecting articulation points on a human by machine learning has been described in the above-described embodiment, the present invention is not limited to such a case. For example, articulation points on a human may be detected by pattern matching using feature points.

Furthermore, although a human or an animal has been described as an example of an object whose fall is to be detected in the above-described example, the present invention is not limited to such a case. Examples of the object may include, in addition to a human and an animal, robots such as an industrial robot and a humanoid robot, and structures or machines having a skeleton.

Furthermore, the means and method for performing various kinds of processing in the above-described fall detection system 100 can be implemented by either a dedicated hardware circuit or a programmed computer. For example, the program may be provided by a computer-readable recording medium such as a USB memory or a digital versatile disc (DVD)-ROM, or may be provided online via a network such as the Internet. In this case, the program recorded on the computer-readable recording medium is usually transferred to and stored in a storage unit such as a hard disk. Furthermore, the program may be provided as independent application software, or may be incorporated in software of a server or another apparatus as one function.

This application is based on Japanese Patent Application No. 2023-084390 filed on May 23, 2023, the disclosure of which is incorporated herein by reference in its entirety. Reference Signs List

- 100: fall detection system
- 200: imaging apparatus
- 201: bus
- 210: controller
- 220: communicator
- 230: camera
- 300: fall detection apparatus
- 301: CPU
- 302: RAM
- 303: ROM
- 304: auxiliary storage unit
- 305: communication I/F
- 306: operation and display unit
- 310: video acquirer
- 320: skeleton detector
- 330: skeleton angle detector
- 340: tracking processor
- 350: primary fall determiner
- 360: secondary fall determiner
- 370: notifier
- 380: UI controller

## Claims

1. A fall detection system comprising:
a video acquirer that acquires a video obtained by capturing an image of an object;
a skeleton detector that detects skeleton information of the object shown in the video;
a skeleton angle calculator that calculates an angle formed by a skeleton of the object with respect to a reference direction, based on the skeleton information of the object;
a primary fall determiner that determines that the object is in a "temporary" fall state when the angle is within a range defined in advance for each skeleton; and
a secondary fall determiner that determines that the object is in a fall state when the determination that the object is in the "temporary" fall state continues for a predetermined first period of time.

2. The fall detection system according to claim 1, wherein the angle is an angle formed by at least one of a skeleton of a body part of the object and a skeleton of a leg part of the object with respect to the reference direction.

3. The fall detection system according to claim 1 or 2, further comprising
a tracking processor that holds same ID information for a same human over time, based on the skeleton information of the object.

4. The fall detection system according to claim 1 or 2, wherein
the skeleton detector estimates a certainty factor as to the skeleton information of the object, and
the skeleton angle calculator calculates the angle when the skeleton information has the certainty factor more than or equal to a predetermined value.

5. The fall detection system according to claim 1 or 2, wherein the primary fall determiner or the secondary fall determiner determines that the object is not in the fall state when the angle satisfies a preset condition although the angle is within the range defined in advance for each skeleton.

6. The fall detection system according to claim 1 or 2, wherein, with regard to the object determined to be in the fall state, when the primary fall determiner subsequently determines that the object is not in the "temporary" fall state for a predetermined second period of time, the secondary fall determiner cancels the determination that the object is in the fall state.

7. The fall detection system according to claim 1 or 2, further comprising:
an imaging apparatus that captures an image of the object and outputs a video including a plurality of the images;
a display device that displays the video shot by the imaging apparatus; and
a display controller that controls the display device,
wherein
the skeleton detector calculates the skeleton information of the object by analyzing an image acquired by the video acquirer, and
the display controller causes the display device to display the skeleton information of the object together with the video.

8. The fall detection system according to claim 7, wherein the display controller makes a color of the object determined to be in the fall state by the secondary fall determiner or a color of the skeleton of the object different from a color of the object not determined to be in the fall state in the video to allow a user to visually distinguish between the object determined to be in the fall state and the object not determined to be in the fall state with ease.

9. The fall detection system according to claim 7, wherein the display controller adds a mark to the object determined to be in the fall state by the secondary fall determiner in the video to allow a user to visually distinguish between the object determined to be in the fall state and the object not determined to be in the fall state with ease.

10. The fall detection system according to claim 9, wherein the display controller keeps the mark added to the object determined to be in the fall state by the secondary fall determiner even when the determination that the object is in the fall state is canceled subsequently.

11. The fall detection system according to claim 7, wherein
the object is a human, and
the display controller subjects the video to obscuring processing or blurring processing at a position of the object so as to prevent the object from being identified, based on position information included in the skeleton information of the object.

12. The fall detection system according to claim 11, wherein the display controller displays the video of the object subjected to the obscuring processing or the blurring processing, with a graphic indicative of the skeleton information of the object superimposed on the video.

13. The fall detection system according to claim 1 or 2, further comprising
a fall notifier that provides a notification of fall of the object when the secondary fall determiner determines that the object is in the fall state.

14. The fall detection system according to claim 13, further comprising
a warning device that outputs an alarm by at least one of sound and light in accordance with the notification from the fall notifier.

15. The fall detection system according to claim 1 or 2, further comprising
a valid area inputter that receives input of a valid area where a determination result of the fall state of the object is valid,
wherein
the primary fall determiner determines the fall state of the object when position coordinates of an articulation point on at least one of a leg part, a head part, and a body part of the object are within the valid area.

16. The fall detection system according to claim 15, further comprising:
an imaging apparatus that captures an image of the object and outputs a video including a plurality of the images;
a display device that displays the video shot by the imaging apparatus; and
a display controller that causes the display device to display a graphic indicative of the valid area together with the video shot by the imaging apparatus.

17. The fall detection system according to claim 1 or 2, further comprising
an invalid area inputter that receives input of an invalid area where a determination result of the fall state of the object is invalid,
wherein
the primary fall determiner does not determine the fall state of the object when position coordinates of an articulation point on at least one of a leg part, a head part, and a body part of the object are within the invalid area.

18. The fall detection system according to claim 17, further comprising:
an imaging apparatus that captures an image of the object and outputs a video including a plurality of the images;
a display device that displays the video shot by the imaging apparatus; and
a display controller that causes the display device to display a graphic indicative of the invalid area together with the video shot by the imaging apparatus.

19. A fall detection method comprising:
a step (a) of acquiring a video obtained by capturing an image of an object;
a step (b) of detecting skeleton information of the object shown in the video;
a step (c) of calculating an angle formed by a skeleton of the object with respect to a reference direction, based on the skeleton information of the object;
a step (d) of determining that the object is in a "temporary" fall state when the angle is within a range defined in advance for each skeleton; and
a step (e) of determining that the object is in a fall state when the determination that the object is in the "temporary" fall state continues for a predetermined first period of time.

20. A fall detection program for causing a computer to execute processing comprising:
a step (a) of acquiring a video obtained by capturing an image of an object;
a step (b) of detecting skeleton information of the object shown in the video;
a step (c) of calculating an angle formed by a skeleton of the object with respect to a reference direction, based on the skeleton information of the object;
a step (d) of determining that the object is in a "temporary" fall state when the angle is within a range defined in advance for each skeleton; and
a step (e) of determining that the object is in a fall state when the determination that the object is in the "temporary" fall state continues for a predetermined first period of time.
